# EUROPÄISCHE PATENTSCHRIFT

(11) **EP 1 731 297 B1**
(45) Veröffentlichungstag und Bekanntmachung des Hinweises auf die Patenterteilung: **25.07.2007**
(21) Anmeldenummer: 06010231.6
(22) Anmeldetag: 18.05.2006
(51) Int. Cl.: B31F 1/28, G01N 33/34

(54) **Wellpappeanlage**
Corrugated board machine
Machine de carton ondulé

(30) Priorität: 08.06.2005 DE 102005026532
(43) Veröffentlichungstag der Anmeldung: 13.12.2006
(73) Patentinhaber: BHS Corrugated Maschinen-und Anlagenbau GmbH, 92729 Weiherhammer (DE)
(72) Erfinder: Baierl, Klaus, 92655 Grafenwöhr (DE); Städele, Norbert, 92711 Parkstein (DE)
(74) Vertreter: Rau, Albrecht

(56) Entgegenhaltungen:
- DE-A1- 19 955 917
- DE-U1- 8 632 116

## Beschreibung

Die Erfindung betrifft eine Wellpappeanlage mit einer Einrichtung zur Erfassung der Qualität der Verleimung zwischen den verschiedenen Material-Bahnen.

Aus der GB 2 369 186 A ist eine Vorrichtung bekannt, um in einer Wellpappeanlage laufend die Qualität der erzeugten Wellpappebahn zu überprüfen. Hierzu wird ein mechanischer Vibrationssensor auf die bewegte Wellpappebahn aufgebracht, der bei dem Transport der Wellpappebahn in Schwingungen versetzt wird. Aus der Art der Schwingungen kann auf die Qualität der Verklebung geschlossen werden. Nachteilig ist hieran, dass der Sensor in ständigem mechanischen Kontakt mit der Wellpappebahn steht, wodurch es an beiden Teilen zu Verschleiß kommen kann. Darüber hinaus ist aufgrund der mechanischen Trägheit des Sensors die Untersuchung der Wellpappebahn bei großen Transportgeschwindigkeiten, beispielsweise 400 m/min, schwierig.

Aus der DE 199 55 917 A1 ist ebenfalls eine Wellpappeanlage mit einer Qualitäts-Erfassungs-Einrichtung bekannt. Bei dieser sind auf beiden Seiten einer einseitig kaschierten Wellpappebahn optische Abstandssensoren angebracht, so dass aus den ermittelten Daten der exakte Verlauf der Wellbahn berechnet werden kann. Hierfür müssen große Datenmengen verarbeitet werden. In Spalte 3, Zeile 31 ff., ist grundsätzlich erwähnt, dass anstelle der zur Abstandsmessung verwendeten Laser-Sensoren auch entsprechende Ultraschall-Sensoren eingesetzt werden können, die die Wellpappe mit Ultraschall abtasten. Wie dies im Einzelnen geschehen soll, ist nicht beschrieben. Darüber hinaus würden auch wieder die Abstände der Außenseite der Wellpappebahn zu den Sensoren bestimmt, so dass wieder große Datenmengen verarbeitet werden müssten.

Der Erfindung liegt die Aufgabe zugrunde, eine Wellpappeanlage zu schaffen, in der die Qualität der Verklebungen in der Wellpappebahn möglichst einfach ermittelt werden kann.

Die Aufgabe wird durch die Merkmale des Anspruches 1 gelöst. Der Kern der Erfindung besteht darin, auf einer Seite der Wellpappebahn einen Ultraschall-Sender anzuordnen und auf der gegenüberliegenden Seite einen Ultraschall-Empfänger. Je besser die Verklebung ist, umso stärker wird der Ultraschall durch die Wellpappebahn übertragen. Ist die Verklebung fehlerhaft, sinkt die Intensität des durch die Pappe übertragenden Ultraschalls stark ab. Das Verfahren kann auch bei Wellpappebahnen verwendet werden, die aus mehr als zwei Materialbahnen bestehen.

Weitere vorteilhafte Ausgestaltungen der Erfindung ergeben sich aus den Unteransprüchen.

Zusätzliche Merkmale und Einzelheiten der Erfindung ergeben sich aus der Beschreibung eines Ausführungsbeispiels anhand der Zeichnung. Es zeigen:
- Fig. 1: einen ersten Teil einer Wellpappeanlage gemäß einem ersten Ausführungsbeispiel,
- Fig. 2: einen zweiten Teil einer Wellpappeanlage gemäß einem ersten Ausführungsbeispiel,

- Fig. 3: eine Draufsicht auf eine Qualitäts-Erfassungs-Einrichtung in der Wellpappeanlage gemäß den Fig. 1 und 2,
- Fig. 4: einen Schnitt gemäß der Schnittlinie IV-IV in Fig. 3,
- Fig. 5: einen Schnitt gemäß der Schnittlinie V-V in Fig. 4,
- Fig. 6: ein vom Ultraschall-Empfänger im Bereich einer fehlerhaften Verleimung produziertes Spannungs-Zeit-Diagramm,
- Fig. 7: eine Fig. 3 entsprechende Darstellung eines zweiten Ausführungsbeispiels,
- Fig. 8: einen Fig. 3 entsprechende Darstellung eines dritten Ausführungsbeispiels, und
- Fig. 9: eine Fig. 5 entsprechende Darstellung eines vierten Ausführungsbeispiels.

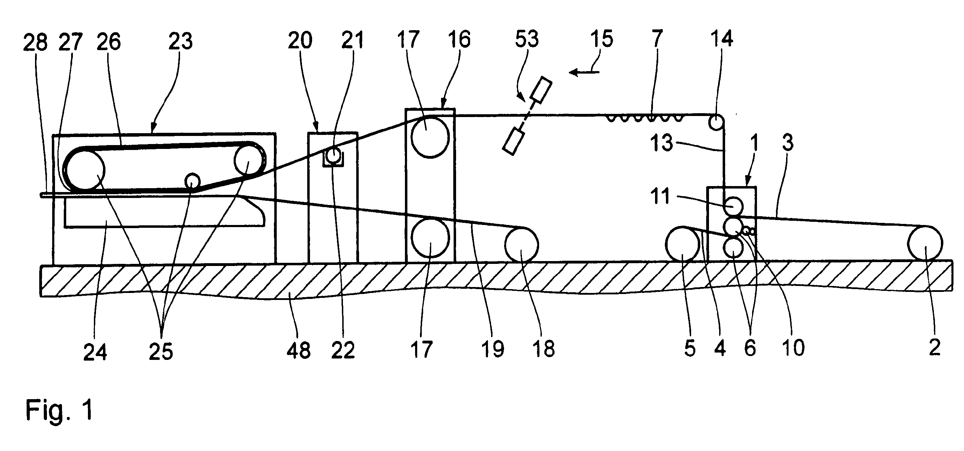

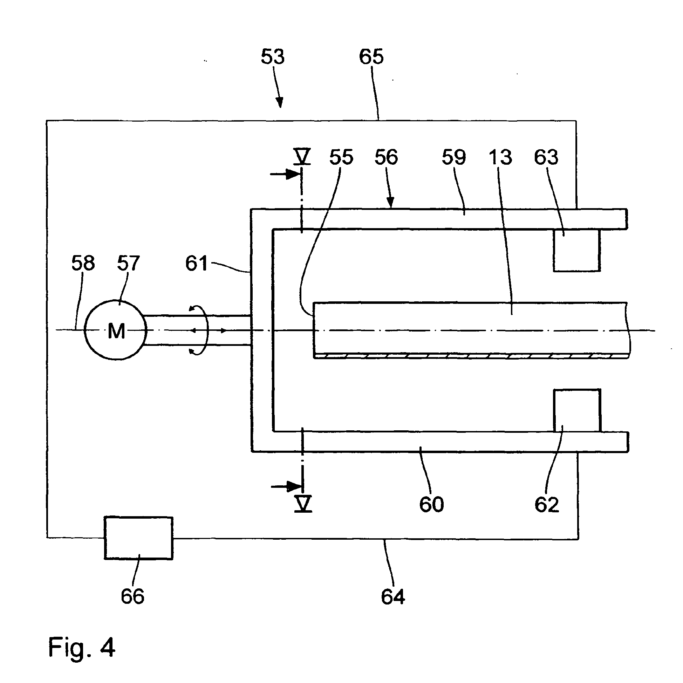

Nachfolgend wird unter Bezugnahme auf die Fig. 1 bis 6 ein erstes Ausführungsbeispiel der Erfindung beschrieben. Eine Wellpappeanlage, wie sie in den Fig. 1 bis 5 schematisch dargestellt ist, weist eine Maschine 1 zur Herstellung einseitig kaschierter Wellpappe auf. Von einer ersten Abroll-Einrichtung 2 wird eine erste Material-Bahn 3 der Maschine 1 zugeführt. Bei den Material-Bahnen handelt es sich um endlose Papierbahnen. Die Material-Bahn 3 stellt eine erste Deckbahn für die in der Maschine 1 hergestellte Wellpappe dar. Die erste Material-Bahn 3 wird in der Maschine 1 mit einer zweiten Material-Bahn 4 zusammengeführt, die von einer zweiten Abroll-Einrichtung 5 abgerollt wird. Nach dem Abrollen wird die zweite Material-Bahn 4 in der Maschine 1 zur Erzeugung einer Wellung zwischen zwei benachbart zueinander angeordneten Riffelwalzen 6 durchgeführt. Die zweite Material-Bahn 4 liegt nach dieser Durchführung als Wellbahn 7 vor. Diese weist in Folge Wellenspitzen 8 und Wellentäler 9 auf. Anschließend wird die Wellbahn 7 in einer Beleimungs-Einrichtung 10 beleimt und die Wellbahn 7 in der Maschine 1 mit der ersten Material-Bahn 3, einer Deckbahn, in einem Spalt zwischen einer Anpress-Walze 11 und einer der Riffelwalzen 6 zusammengedrückt und miteinander verbunden, wodurch eine Verleimung 12 entsteht. Die entstehende, aus Deckbahn 3 und Wellbahn 7 bestehende, einseitig kaschierte Wellpappebahn 13 wird nach oben abgeführt und um eine Umlenkwalze 14 in eine Arbeits-Richtung 15 umgelenkt. Die Maschine 1 zur Herstellung einseitig kaschierter Wellpappebahnen ist allgemein bekannt, beispielsweise aus der EP 0 687 552 A (entspr. US-Patent 5,632,850), der DE 195 36 007 A (entspr. GB 2,305,675 A) oder der DE 43 05 158 A, worauf bezüglich der Einzelheiten verwiesen wird.

Der Maschine 1 in Arbeits-Richtung 15 nachgeordnet ist eine Vorheiz-Einrichtung 16. Diese weist zwei übereinander angeordnete, beheizbare Heiz-Walzen 17 auf. Unmittelbar vor der Vorheiz-Einrichtung 16 ist eine zweite Abroll-Einrichtung 18 für eine dritte Material-Bahn 19 angeordnet, von der diese abgewickelt und entlang der Arbeits-Richtung 15 durch die Vorheiz-Einrichtung 16 transportiert wird. Die einseitige Wellpappebahn 13 und die dritte Material-Bahn 19 umschlingen beide teilweise die Heiz-Walzen 17 und werden entlang der Richtung 15 an diesen vorbeigeführt. In Richtung 15 hinter der Vorheiz-Einrichtung 16 ist ein Leimwerk 20 mit einer Beleimungs-Walze 21 angeordnet, die teilweise in ein Leimbad 22 eintaucht. Die Wellbahn 7 der Wellpappebahn 13 befindet sich in Kontakt mit der Beleimungs-Walze 21.

Hinter dem Leimwerk 20 ist eine Heiz-Andrück-Einrichtung 23 angeordnet, die einen horizontalen, sich in Arbeits-Richtung 15 erstreckenden Tisch 24 mit Heizplatten aufweist. Oberhalb des Tisches 24 ist ein über drei Walzen 25 umgelenkter, angetriebener, endloser Andruck-Gurt 26 vorgesehen. Zwischen dem Andruck-Gurt 26 und dem Tisch 24 ist ein Anpressspalt 27 gebildet, durch den die Wellpappebahn 13 und die dritte Material-Bahn 19 geführt sind und dort aneinander gedrückt werden. Eine entsprechende Heiz-Andrück-Einrichtung 23 ist aus der DE 199 54 754 A bekannt. In der Heiz-Andrück-Einrichtung 23 wird eine dreilagige Wellpappebahn 28 gebildet.

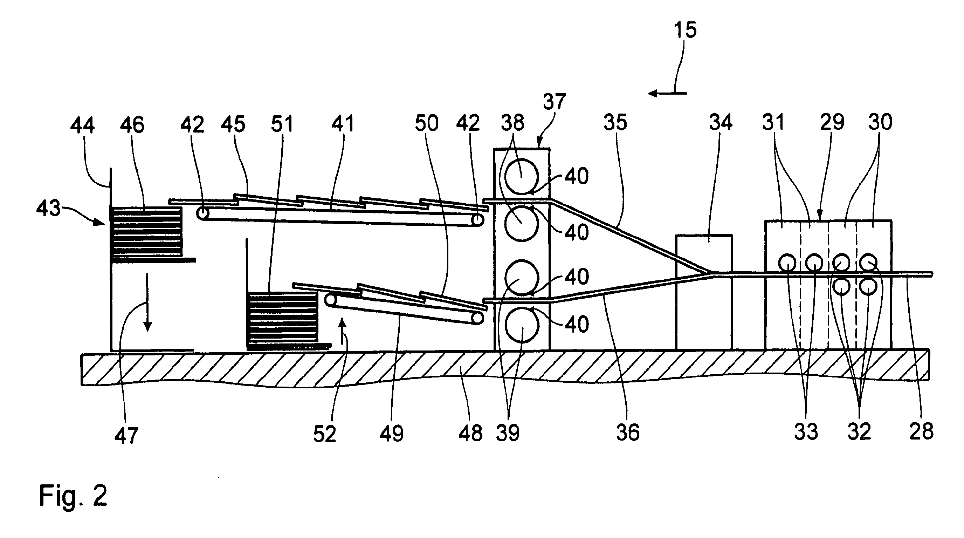

Fig. 2 zeigt einen zweiten Teil der Wellpappeanlage im Anschluss an den Austritt der Wellpappebahn 28 aus der Heiz-Andrück-Einrichtung 23. Es folgt eine Längsschneide-Rill-Einrichtung 29, die aus zwei hintereinander angeordneten Rill-Stationen 30 sowie zwei hintereinander angeordneten Längsschneide-Stationen 31 besteht. Die Rill-Stationen 30 weisen jeweils paarweise übereinander angeordnete Rillwerkzeuge 32 auf, zwischen denen die Wellpappebahn 28 durchgeführt ist. Die Längsschneide-Stationen 31 weisen jeweils drehantreibbare Messer 33 auf, die mit der Wellpappebahn 28 zur Längsdurchtrennung derselben in Eingriff bringbar sind. Der genauere Aufbau der Längsschneide-Rill-Einrichtung 29 ist aus der DE 197 54 799 A (entspr. US 6,071,222) sowie der DE 101 31 833 A bekannt, auf die hiermit für den Detailaufbau verwiesen wird. In Arbeits-Richtung hinter der Längsschneide-Rill-Einrichtung 29 ist eine Weiche 34 angeordnet, in der längsgeschnittene Bahnabschnitte 35, 36 der Wellpappebahn 28 voneinander getrennt werden. Die Bahnabschnitte 35, 36 werden nachfolgend einer Querschneide-Einrichtung 37 zugeführt. Diese weist für den oberen Bahnabschnitt 35 ein oberes Querschneide-Walzenpaar 38 und für den unteren Bahnabschnitt 36 ein unteres Querschneide-Walzenpaar 39 auf. Die Walzen der Walzenpaare 38, 39 tragen jeweils ein sich radial nach außen erstreckendes und senkrecht zur Arbeits-Richtung 15 verlaufendes Messer 40. Die Messer 40 eines Querschneide-Walzenpaares 38, 39 wirken zur Querdurchtrennung der Bahnabschnitte 35, 36 zusammen. Dem oberen Querschneide-Walzenpaar 38 ist ein oberes Förderband 41 nachgeordnet, welches um drehantreibbare Walzen 42 umgelenkt wird. Hinter dem oberen Förderband 41 ist eine Ablage 43 mit vertikal verlaufendem Anschlag 44 angeordnet, auf der aus dem Bahnabschnitt 35 mittels der Querschneide-Einrichtung 37 geschnittene Wellpappe-Bögen 45 einen Stapel 46 bildend gestapelt werden. Die Ablage 43 ist, wie durch einen Richtungspfeil 47 angedeutet, in der Höhe verstellbar. Insbesondere kann die Ablage 43 zum Weitertransport des Stapels 46 bis zu einem Maschinenboden 48, der die Wellpappeanlage trägt, abgesenkt werden. Dem unteren Querschneide-Walzenpaar 39 ist ein weiteres, unteres Förderband 49 nachgeordnet, das Wellpappe-Bögen 50, die mittels der Querschneide-Einrichtung 37 aus dem Bahnabschnitt 36 geschnitten wurden, auf einer weiteren Ablage 51 stapelt. Zur Anpassung an die Höhe des Stapels 46 kann das untere Förderband 49 angehoben werden, wie durch einen Richtungspfeil 52 angedeutet.

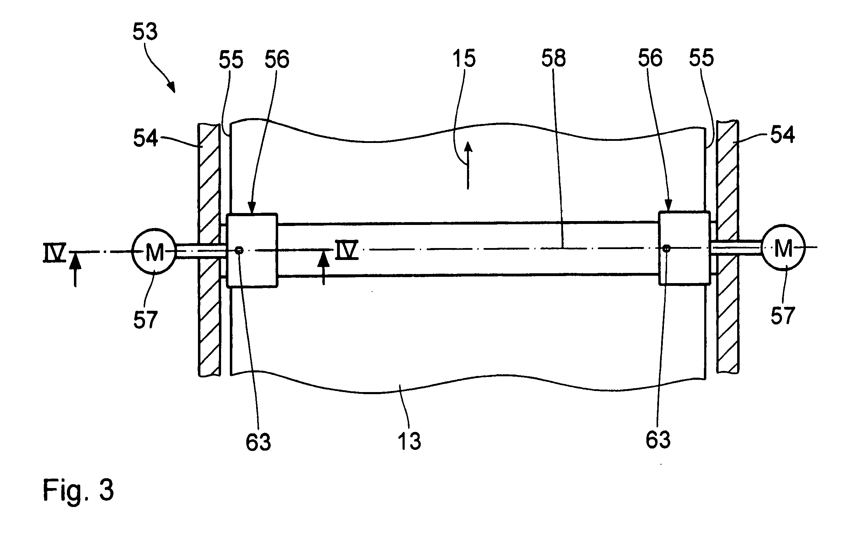

Die Wellpappeanlage weist eine in den Fig. 3 bis 5 näher dargestellte Qualitäts-Erfassungs-Einrichtung 53 auf. Diese dient der Erfassung der Qualität der Verleimungen 12 in der einseitig kaschierten Wellpappebahn 13. Insofern ist die Einrichtung 53 in Arbeits-Richtung 15 hinter der Maschine 1 und vor der Heiz-Andrück-Einrichtung 23 angeordnet, in der die dritte Materialbahn 19 angedrückt wird. Bevorzugt ist die Platzierung zwischen der Maschine 1 und der Vorheiz-Einrichtung 16. Die Einrichtung 53 weist auf dem Maschinenboden 48 auf beiden Seiten der Wellpappebahn 13 abgestützte Träger 54 auf. An beiden Trägern 54 ist jeweils ein den jeweiligen Rand 55 der Wellpappebahn 13 umgreifender, im Querschnitt U-förmiger Träger 56 angeordnet, der gegenüber dem Träger 54 abgestützt ist. Außerhalb des Trägers 54 ist jeweils ein Antrieb 57 vorgesehen, der eine Verschwenkung um eine Schwenkachse 58 ermöglicht. Die Schwenkachse 58 liegt mittig in der Wellpappebahn 13 und verläuft senkrecht zur Arbeits-Richtung 15. Der Antrieb 57 erlaubt ferner eine Verschiebung des Trägers 56 entlang der Schwenkachse 58, so dass der U-förmige Träger 56 auch bei kleineren Breiten der Wellpappebahn 13 um den Rand 55 geschoben werden kann. Der Träger 56 weist zwei parallel zueinander verlaufende Schenkel 59, 60 auf, die durch eine gemeinsame Basisplatte 61, die senkrecht zu diesen verläuft, miteinander verbunden und einteilig damit ausgebildet sind. Auf der Innenseite des Schenkels 60 ist ein Ultraschall-Sender 62 angeordnet. Auf der gegenüberliegenden Innenseite des Schenkels 59 ist ein zugehöriger Ultraschall-Empfänger 63 angeordnet. Der Sender 62 und der Empfänger 63 weisen in Bezug auf die Wellpappebahn 13 dieselbe Transversal-Position auf, d. h. sie haben denselben senkrechten Abstand vom Rand 55 der Wellpappebahn 13. Der Sender 62 ist über eine Leitung 64 und der Empfänger 63 über eine Leitung 65 mit einer gemeinsamen Steuer-Auswerte-Einheit 66 in datenübertragender Weise verbunden.

Der Sender 62 und der Empfänger 63 liegen auf einer gemeinsamen Mittel-Längs-Geraden 67. Die zweite Materialbahn 4 liegt in einer diese definierenden Ebene 68. Die Gerade 67 schließt mit der Ebene 68 einen Winkel b ein. Durch die Verschwenkbarkeit des Trägers 56 um die Achse 58 ist der Winkel b einstellbar.

Zwischen jeweils einer Wellenspitze 8 und einem Wellental 9 befindet sich eine Flanke 69, die mit der Ebene 68 einen Winkel c einschließt. Der Winkel b wird so gewählt, dass er nach Möglichkeit dem Winkel c entspricht.

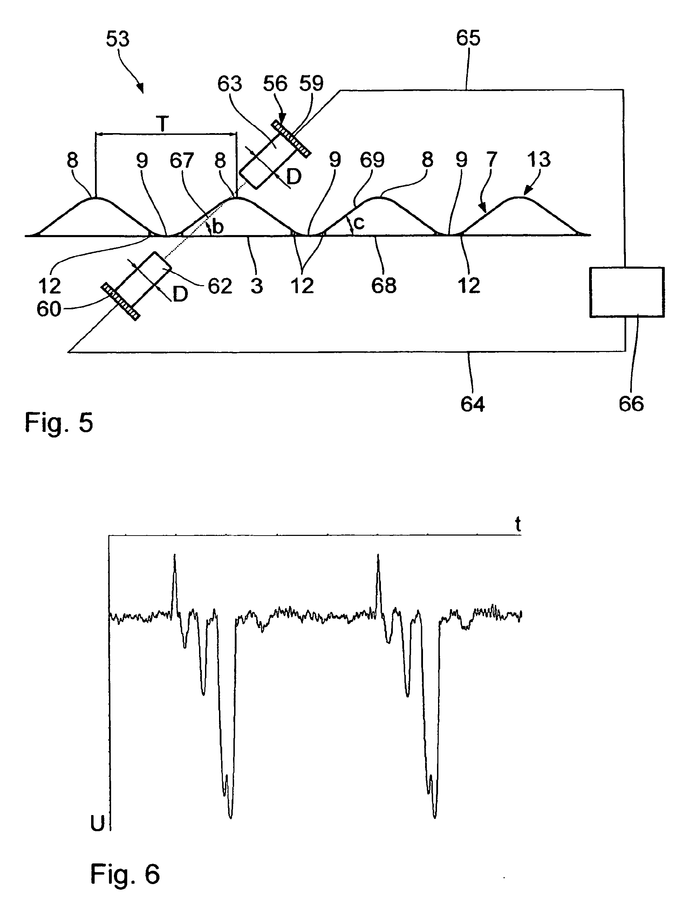

Dies bedeutet, dass der Ultraschall nach Möglichkeit so weit wie möglich durch die Flanke 69 der Wellbahn 7 selbst und nicht durch die sie umgebende Luft übertragen wird. Für den Winkel b gilt grundsätzlich:
0° ≤b ≤90°, insbesondere 0° < b < 90°, insbesondere 15° ≤b ≤65°, insbesondere 35° ≤b ≤45°, insbesondere b ≈40°. Durch die Verschwenkbarkeit des Trägers 56 kann der Winkel b an den Flankenwinkel c für verschiedene Wellpappentypen angepasst werden. Der Ultraschall-Sender 62 arbeitet beispielsweise bei einer Frequenz von ca. 200 kHz. Die Darstellung in Fig. 5 ist insofern nicht maßstabsgetreu, als die Wellpappebahn 13 überproportional vergrößert dargestellt ist. Grundsätzlich weist die Wellpappebahn 13 eine Teilung T auf. Der Sender 62 und der Empfänger 63 weisen jeweils einen Durchmesser D auf. Bevorzugterweise soll der Durchmesser D größer als die Teilung T sein. Typische Durchmesser D sind 16 mm oder 20 mm. Die Positionen von Sender 62 und Empfänger 63 können auch vertauscht werden. Darüber hinaus ist es möglich, eine Anordnung vorzusehen, die sich daraus ergibt, wenn man Sender 62 und Empfänger 63 um einen Winkel von 180° - 2b um den Schnittpunkt zwischen der Geraden 67 und der Ebene 68 gegen den Uhrzeigersinn dreht. Hierdurch wird die stromaufwärtige Position des Senders bzw. Empfängers durch eine entsprechende stromabwärtige Position getauscht und umgekehrt. Es bleibt jedoch bei dem betragsmäßigen Winkel b, der nun links von der Geraden 67 und nicht rechts von dieser, wie in Fig. 5, gemessen wird.

Im Folgenden wird unter Bezugnahme auf Fig. 6 die Funktionsweise der Qualitäts-Erfassungs-Einrichtung 53 beschrieben. Sobald die Maschine 1 die einseitig kaschierte Wellpappebahn 13 erzeugt hat, wird diese mit vorbekannter Geschwindigkeit durch die Einrichtung 53 geführt. Hierbei sind die Träger 56 bezüglich ihrer Transversalposition beide so eingestellt, dass sie die beiden Ränder 55 der Wellpappebahn 13 umgreifen, aber nicht berühren. Der Ultraschall-Sender 62 sendet konstant Ultraschallwellen aus, die vom zugehörigen Ultraschall-Empfänger 63 empfangen werden. Aufgrund der Tatsache, dass Ultraschall durch Masse, d. h. durch Papier, wesentlich besser übertragen wird, als durch Luft, entsteht im Fall perfekter Verleimungen 12 ein pulsierendes Signal, je nach dem, ob das Signal, wie im Fall von Fig. 5, gerade wesentlich durch die Flanke 69 übertragen wird oder kurz danach wesentlich durch Luft übertragen werden muss. Wäre der Winkel b = 90°, so wären die Unterschiede in der Signalintensität am Empfänger 63 zwischen einer gerade durchlaufenden Wellenspitze 8 und einem Wellental 9 sehr gering, da in beiden Fällen der Ultraschall wesentlich durch Luft, sei es zwischen der Materialbahn 3 und der Wellenspitze 8 oder oberhalb des Wellentales 9 übertragen werden müsste. In diesem Fall wäre die Signalauswertung außerordentlich kompliziert. Durch die Neigung des Trägers 56 um einen Winkel b < 90°, der im Wesentlichen dem Flankenwinkel c entspricht, wird erreicht, dass beim Durchlauf der Wellenspitze 8 das Ultraschallsignal im Wesentlichen durch die Flanke 69, die Verleimung 12 und die Materialbahn 3 übertragen wird, so dass bei einwandfreier Verleimung 12 ein besonders großes Signal entsteht, das beim Weitertransport der Wellpappebahn 13 entsprechend stark abfällt.

Liegt eine fehlerhafte Verleimung 12 vor oder ist es zu einem sogenannten Flankenbruch gekommen, so liegt an der Stelle, an der ein großes Signal zu erwarten gewesen wäre, ein Signal sehr viel niedrigerer Intensität vor. Dieses Signal kann durch Vorgabe bestimmter Schwellwerte einfach in ein digitales Signal umgewandelt werden, wobei "1" Produktionsfehler bedeutet und "0" Fehlerfreiheit bedeutet. Fig. 6 zeigt den starken Spannungsabfall bei dem Durchlauf zweier fehlerhafter Verleimungen. Vorteilhaft an der Einrichtung 53 ist, dass diese berührungslos und trägheitslos arbeitet. Für die einfache Schwellwertanalyse der Signale des Empfängers 63 ist kein großer elektronischer Aufwand erforderlich, so dass auch bei sehr großen Materialbahngeschwindigkeiten, z. B. 400 m/min, eine Onlineerfassung der Verleimungsqualität möglich ist.

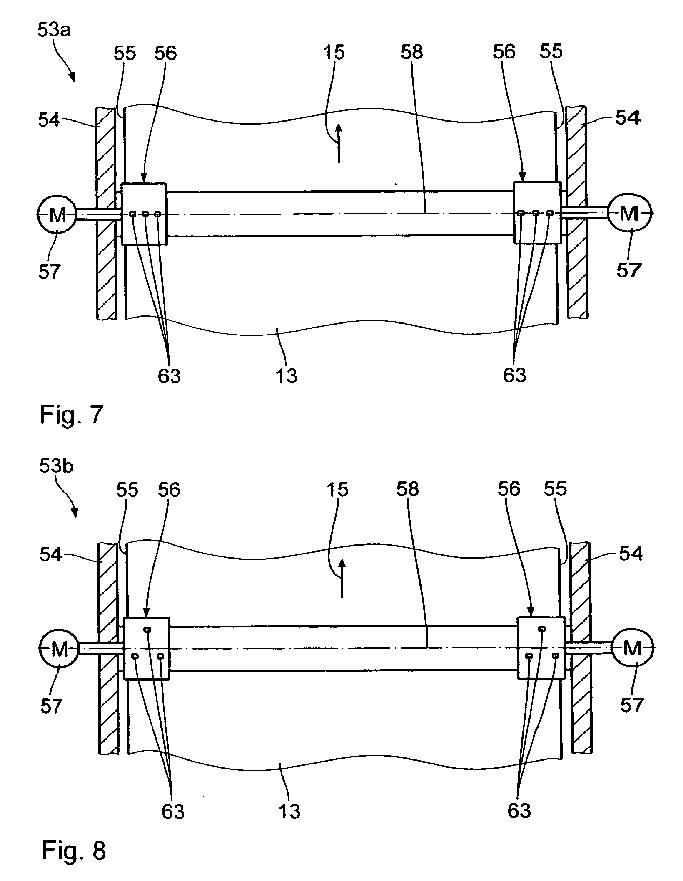

Im Folgenden wird unter Bezugnahme auf Fig. 7 ein zweites Ausführungsbeispiel der Erfindung beschrieben. Konstruktiv identische Teile erhalten dieselben Bezugszeichen wie beim ersten Ausführungsbeispiel, auf dessen Beschreibung hiermit verwiesen wird. Konstruktiv unterschiedliche, jedoch funktionell gleichartige Teile erhalten dieselben Bezugszeichen mit einem nachgestellten a. Der wesentlichen Unterschied gegenüber dem ersten Ausführungsbeispiel besteht darin, dass nebeneinander mehrere Sender 62 und entsprechend gegenüberliegend mehrere Empfänger 63 in einer Linie angeordnet sind. Dies hat den Vorteil, dass die Qualität der Verleimung 12 über eine größere Breite untersucht werden kann. Grundsätzlich ist es möglich, die Schenkel 59 und 60 so lang auszubilden, dass beide Träger 56 insgesamt die gesamte Wellpappebahn 13 umgreifen. Dies gilt für alle Ausführungsbeispiele. Ferner ist es möglich, die Sender 62 und die zugeordneten Empfänger 63 auf einem transversal, d. h. quer zur Arbeits-Richtung 15, verfahrbaren Schlitten anzuordnen. Auf die Weise ist es ebenfalls möglich, die Qualität der Verleimung 12 über die gesamte Breite der Wellpappebahn 13 zu untersuchen.

Im Folgenden wird unter Bezugnahme auf Fig. 8 ein drittes Ausführungsbeispiel der Erfindung beschrieben. Identische Teile erhalten dieselben Bezugszeichen wie beim ersten Ausführungsbeispiel. Konstruktiv unterschiedliche, jedoch funktionell gleichartige Teile erhalten dieselben Bezugszeichen mit einem nachgestellten b. Der wesentliche Unterschied gegenüber dem zweiten Ausführungsbeispiel besteht darin, dass auch mehrere Sender 62 und mehrere zugeordnete Empfänger 63 angeordnet sind, diese jedoch nicht auf einer Linie angeordnet sind, sondern im Zick-Zack. Dies hat den Vorteil, dass bei einer vorbestimmten Länge der Schenkel 59, 60 quer zur Arbeits-Richtung 15 mehr Sender 62 bzw. Empfänger 63 und diese auch dichter angeordnet werden können. Für jeden Sender 62 mit einem vorbestimmten Durchmesser D ist ein Mindestabstand zum nächsten benachbarten Sender erforderlich, damit die Signale sich auf der Empfangsseite nicht überlappen. Durch die Anordnung gemäß Fig. 8 können mehr Sender bei vorgegebener Schenkellänge untergebracht werden, so dass auch eine genauere Analyse der Verleimungsqualität möglich ist.

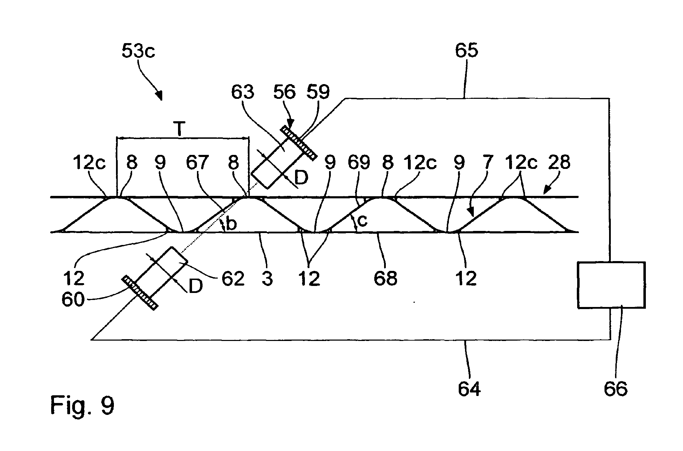

Im Folgenden wird unter Bezugnahme auf Fig. 9 ein viertes Ausführungsbeispiel der Erfindung beschrieben. Konstruktiv identische Teile erhalten dieselben Bezugszeichen wie beim ersten Ausführungsbeispiel, auf dessen Beschreibung hiermit verwiesen wird. Konstruktiv unterschiedliche, jedoch funktionell gleichartige Teile erhalten dieselben Bezugszeichen mit einem nachgestellten c. Der wesentliche Unterschied gegenüber dem ersten Ausführungsbeispiel besteht darin, dass die Qualitäts-Erfassungs-Einrichtung 53c der Qualitätserfassung einer aus drei Material-Bahnen oder mehr bestehenden Wellpappebahn 28 dient. Entsprechend ist die Einrichtung 53c in Arbeits-Richtung 15 hinter der Heiz-Andrück-Einrichtung 23 und vor der Querschneide-Einrichtung 37 angeordnet. Bevorzugt ist eine Anordnung zwischen der Längsschneide-Rill-Einrichtung 29 und der Weiche 34. Die Einrichtung 53c ist im Wesentlichen so aufgebaut wie die Einrichtung 53 gemäß dem ersten Ausführungsbeispiel. Lediglich die zu untersuchende Wellpappebahn 28 weist mehr Materialbahnen auf. Es sind insofern mehrere Verleimungen 12, 12c auf Qualität zu untersuchen. Grundsätzlich ist die Funktionsweise der Fehlererkennung dieselbe. Sind Verleimungen 12, 12c fehlerhaft, so ist die Übertragung der Ultraschallsignale dort geringer als bei einer perfekten Verleimung. Die Einrichtung 53c kann auch zur Untersuchung von Wellpappebahnen mit weiteren Materialbahnen, beispielsweise einer Wellpappebahn mit drei glatten Materialbahnen und zwei Wellbahnen verwendet werden.

## Patentansprüche

1. Anlage zur Herstellung einer entlang einer Arbeits-Richtung (15) transportierten Wellpappebahn (13, 28)
a. mit mindestens einer glatten Deckbahn (3, 19), und
b. mit mindestens einer gewellten, Wellenspitzen (8) und Wellentäler (9) aufweisenden Wellbahn (7),
c. wobei die Wellbahn (7) zumindest im Bereich der Wellentäler (9) mit der mindestens einen Deckbahn (3) verleimt ist, **dadurch gekennzeichnet, dass**
d. eine Qualitäts-Erfassungs-Einrichtung (53; 53a; 53b; 53c) zur Erfassung der Qualität der Verleimung zwischen der mindestens einen Deckbahn (3, 19) und der mindestens einen Wellbahn (7) vorgesehen ist, welche aufweist
i. mindestens einen auf einer Seite der Wellpappebahn (13, 28) angeordneten Ultraschall-Sender (62),
ii. mindestens einen auf der gegenüberliegenden Seite der Wellpappebahn (13, 28) angeordneten, dem mindestens einen Ultraschall-Sender (62) zugeordneten Ultraschall-Empfänger (63), und
iii. eine mit dem mindestens einen Ultraschall-Empfänger (63) in datenübertragender Weise verbundene Steuer-AuswerteEinrichtung (66) zur Auswertung des Signals des Ultraschall-Empfängers (63).

2. Anlage gemäß Anspruch 1, **dadurch gekennzeichnet, dass** der mindestens eine Ultraschall-Sender (62) und der mindestens eine Ultraschall-Empfänger (63) bezogen auf die Arbeits-Richtung (15) der Wellpappebahn (13, 28) dieselbe Transversal-Position besitzen.

3. Anlage gemäß Anspruch 1 oder 2, **dadurch gekennzeichnet, dass** der mindestens eine Ultraschall-Sender (62) und der mindestens eine Ultraschall-Empfänger (63) auf einer Gerade (67) liegen, die mit der Arbeits-Richtung (15) einen Winkel (b) einschließt, fiir den gilt: 0° < b < 90°, insbesondere 35° ≤b ≤45°, insbesondere b ≈40°.

4. Anlage gemäß Anspruch 3, **dadurch gekennzeichnet, dass** die Wellbahn (7) zwischen jeder Wellenspitze (8) und jedem Wellental (9) eine Flanke (69) mit einem Steigungswinkel (c) besitzt und ferner gilt: b ≈c.

5. Anlage gemäß einem der vorangehenden Ansprüche, **dadurch gekennzeichnet, dass** die Wellbahn (7) eine Teilung (T) aufweist und der Ultraschall-Sender (62) und/oder der Ultraschall-Empfänger (63) entlang der Arbeits-Richtung (15) einen Durchmesser (D) aufweisen, wobei gilt: D ≥T.

6. Anlage gemäß einem der vorangehenden Ansprüche, **dadurch gekennzeichnet, dass** der mindestens eine Ultraschall-Sender (62) und der mindestens eine Ultraschall-Empfänger (63) quer zur Arbeits-Richtung (15) verfahrbar sind.

7. Anlage gemäß einem der vorangehenden Ansprüche, **dadurch gekennzeichnet, dass** quer zur Arbeits-Richtung (15) mehrere Ultraschall-Sender (62) und/oder Ultraschall-Empfänger (63) angeordnet sind.

8. Anlage gemäß Anspruch 7, **dadurch gekennzeichnet, dass** die Ultraschall-Sender (62) und/oder die Ultraschall-Empfänger (63) auf einer Zick-Zack-Linie angeordnet sind.

9. Anlage gemäß einem der vorangehenden Ansprüche, **dadurch gekennzeichnet, dass** der mindestens eine Ultraschall-Sender (62) und der mindestens eine Ultraschall-Empfänger (63) auf einem gemeinsamen, um eine horizontale Schwenkachse (58) verschwenkbaren Träger (56) angeordnet sind.

10. Anlage gemäß einem der vorangehenden Ansprüche, **dadurch gekennzeichnet, dass** die Wellpappebahn (28) eine mit der Wellbahn (7) verklebte erste Deckbahn (3) und eine auf der anderen Seite der Wellbahn (7) angeordnete, mit dieser verklebte zweite Deckbahn (19) aufweist.

## Claims

1. A machine for the manufacture of a web of corrugated board (13, 28) which is conveyed in a working direction (15), comprising
a. at least one smooth liner (3, 19); and
b. at least one corrugated medium (7) which has corrugation tips (8) and corrugation troughs (9);
c. with the corrugated medium (7) and the at least one liner (3) being assembled by adhesion at least in the vicinity of the corrugation troughs (9); **characterized in that**
d. a quality assessment device (53; 53a; 53b; 53c) for assessment of the quality of the adhesive bond between the at least one liner (3, 19) and the at least one corrugated medium (7) is provided, comprising
i. at least one ultrasonic transmitter (62) disposed on one side of the web of corrugated board (13,28);
ii. at least one ultrasonic receiver (63) disposed on the opposite side of the web of corrugated board (13,28) and allocated to the at least one ultrasonic transmitter (62); and
iii. a control and evaluation unit (66) which, in a manner for data transmission, is connected to the at least one ultrasonic receiver (63) for evaluation of the signal of the ultrasonic receiver (63).

2. A machine according to claim 1, **characterized in that** the at least one ultrasonic transmitter (62) and the at least one ultrasonic receiver (63) have the same transversal position as related to the working direction (15) of the web of corrugated board (13, 28).

3. A machine according to claim 1 or 2, **characterized in that** the at least one ultrasonic transmitter (62) and the at least one ultrasonic receiver (63) are located on a straight line (67) which cooperates with the working direction (15) to make an angle (b) to which applies 0° < b< 90°, in particular 35° ≤ b≤ 45°, in particular b≈ 40°.

4. A machine according to claim 3, **characterized in that** the corrugated medium (7), between each corrugation tip (8) and each corrugation trough (9), possesses a flank (69) with an angle of slope (c) and that b ≈ c applies.

5. A machine according to one of the preceding claims, **characterized in that** the corrugated medium (7) has a spacing (T) and the ultrasonic transmitter (62) and/or the ultrasonic receiver (63) has a diameter (D) in the working direction (15), with D ≥ T applying.

6. A machine according to one of the preceding claims, **characterized in that** the at least one ultrasonic transmitter (62) and the at least one ultrasonic receiver (63) are movable transversely to the working direction (15).

7. A machine according to one of the preceding claims, **characterized in that** several ultrasonic transmitters (62) and/or ultrasonic receivers (63) are disposed transversely to the working direction (15).

8. A machine according to claim 7, **characterized in that** the ultrasonic transmitters (62) and/or the ultrasonic receivers (63) are disposed on a zigzag line.

9. A machine according to one of the preceding claims, **characterized in that** the at least one ultrasonic transmitter (62) and the at least one ultrasonic receiver (63) are disposed on a joint support (56) which is pivotable about a horizontal pivoting axis (58).

10. A machine according to one of the preceding claims, **characterized in that** the web of corrugated board (28) comprises a first liner (3), which is applied by adhesion to the corrugated medium (7), and a second liner (19), which is disposed on the other side of the corrugated medium (7) and applied thereto by adhesion.

## Revendications

1. Dispositif pour la fabrication d'une bande de carton ondulé (13, 28) transportée dans un sens de travail (15)
a. avec au moins une bande de couverture (3, 19) et
b. avec au moins une bande ondulée (7) présentant des crêtes (8) et des ressacs (9),
c. où la bande ondulée (7), du moins au niveau des ressacs (9), est collée avec au moins une bande de couverture (3), **caractérisé en ce que**
d. un dispositif de reconnaissance de qualité (53, 53a, 53b, 53c) pour l'enregistrement de la qualité du collage entre au moins une bande de couverture (3, 19) et au moins une bande ondulée (7), est prévu, lequel présente
i. au moins un émetteur d'ultrasons (62) placé sur un côté de la bande de carton ondulé (13, 28),
ii. au moins un récepteur d'ultrasons (63) placé sur le côté opposée de la bande de carton ondulé (13, 28) et affecté à au moins un émetteur d'ultrasons (62)
iii. une unité d'évaluation de direction (66), pour l'évaluation du signal du récepteur d'ultrasons (63), relié à au moins un récepteur d'ultrasons (63) pour la transmission de données.

2. Dispositif selon la revendication 1, **caractérisé en ce que**
au moins un émetteur d'ultrasons (62) et au moins un récepteur d'ultrasons (63), ont, par rapport au sens de travail (15) de la bande de carton ondulé (13, 28), la même position transversale.

3. Dispositif selon la revendication 1 ou 2, **caractérisé en ce que** au moins un émetteur d'ultrasons (62) et au moins un récepteur d'ultrasons (63) sont sur une droite (67), qui forme un angle (b) avec le sens de direction (15), pour lequel on a : 0° < b < 90°, en particulier 35°≤ b ≤ 45°, en particulier b = 40°.

4. Dispositif selon la revendication 3, **caractérisé en ce que** la bande ondulée (7) possède un flanc (69) entre chaque crête (8) et chaque ressac (9) avec un angle d'inclinaison et on a en outre : b=c.

5. Dispositif selon l'une des revendications précédentes, **caractérisé en ce que** la bande ondulée (7) présente une division (T) et l'émetteur d'ultrasons (62) et/ou le récepteur d'ultrasons (63) le long du sens de travail (15), présentent un diamètre (D), où on a : D ≥ T.

6. Dispositif selon l'une des revendications précédentes, **caractérisé en ce que** au moins un émetteur d'ultrasons (62) et au moins un récepteur d'ultrasons (63) peuvent être déplacés transversalement au sens de travail (15).

7. Dispositif selon l'une des revendications précédentes, **caractérisé en ce que** plusieurs émetteurs d'ultrasons (62) et/ou récepteurs d'ultrasons (63) sont disposés transversalement au sens de travail (15).

8. Dispositif selon la revendication 7, **caractérisé en ce que** les émetteurs d'ultrasons (62) et/ou les récepteurs d'ultrasons (63) sont disposés sur une ligne en zigzags.

9. Dispositif selon l'une des revendications précédentes, **caractérisé en ce que** au moins un émetteur d'ultrasons (62) et au moins un récepteur d'ultrasons (63) sont disposés ensemble sur un support pivotant (56) à l'horizontale d'un axe pivotant (58).

10. Dispositif selon l'une des revendications précédentes, **caractérisé en ce que** la bande de carton ondulé (28) présente une première bande de couverture (3) collée avec la bande ondulée (7), et une seconde bande de couverture (19) disposée sur l'autre côté de la bande ondulée (7) et collée à celle-ci.
